# EUROPÄISCHE PATENTSCHRIFT

(11) **EP 0 448 961 B1**
(45) Veröffentlichungstag und Bekanntmachung des Hinweises auf die Patenterteilung: **02.08.1995**
(21) Anmeldenummer: 91102666.4
(22) Anmeldetag: 23.02.1991
(51) Int. Cl.: A61K 31/21, A61K 9/12

(54) **Nitroglycerin-Pumpspray**
Nitroglycerine pumpspray
Aérosol de nitroglycérine

(30) Priorität: 10.03.1990 DE 4007705
(43) Veröffentlichungstag der Anmeldung: 02.10.1991
(73) Patentinhaber: G. Pohl-Boskamp GmbH & Co. Chemisch-pharmazeutische Fabrik, 25551 Hohenlockstedt (DE)
(72) Erfinder: Cholcha, Walter, Dr., W-2200 Elmshorm (DE)
(74) Vertreter: UEXKÜLL & STOLBERG

(56) Entgegenhaltungen:
- EP-A- 0 310 910
- DE-A- 3 246 081
- FR-A- 2 633 933

## Beschreibung

Die Erfindung betrifft ein Nitroglycerin-Pumpspray mit einem Gehalt an 0,2 bis 3,5 Gew.% Nitroglycerin und bis zu 3 Gew.% üblicher Zusatzstoffe wie Geschmacksstoff oder Antioxydantien.

Nitroglycerin-Präparate bzw. Glyceroltrinitrat (GTN) werden zur Kupierung und Vorbeugung von Angina-pectoris-Anfällen, bei Myocardinfarkten, bei Lungenödem, Asthma cardiale, Koronarsklerose, bei spastischer Migräne und spastischen Gallenwegskoliken vorzugsweise sublingual eingesetzt und führen zu einer prompten Wirkung auf die Schmerzen bzw. kritischen Herz-Kreislaufzustände mit Minderdurchblutung und mit erhöhtem Druck in den Herzkammern.

Bei versprühbaren sublingual anzuwendenden Aerosol-Sprays hat man bislang Zubereitungen verwendet, die neben Nitroglycerin und üblichen Zusätzen ein Neutralöl als Trägerstoff und 70 bis 95 Gew.% Treibgas wie Dichlortetrafluoromethan enthalten und beispielsweise in der DE-PS 32 46 081 beschrieben sind.
Diese nitroglycerinhaltigen Aerosol-Präparate gewährleisten zwar einen therapeutisch wirksamen Blutspiegel in kurzer Zeit, haben jedoch den Nachteil, daß sie auf Grund der vorhandenen FCKW-Treibgase umweltschädigend sein können.

Demzufolge wurden treibgasfreie Aerosol-Präparate, nämlich sogenannte Pumpsprays z.B. gemäß DE-OS 39 22 650 vorgeschlagen, bei denen die flüssige Phase aus aliphatischen C₂- bis C₄-Alkoholen und einem Trägerstoff besteht. Bei diesen Nitroglycerin-Pumpsprays soll der C₂- bis C₄-Alkohlgehalt 51 bis 90 Gew.% betragen, und ferner soll als Trägerstoff ein Polyalkylenglykol oder ein mehrere Hydroxygruppen aufweisender C₂- bis C₈-Alkohol vorgesehen werden.

Derartige Zubereitungen haben den Nachteil, daß einmal aufgrund des hohen Alkohlgehalts die in der Dosierkammer des Pumpspray-Gerätes vorhandene Ausbringungsmenge von etwa 50 mg schon bei kurzzeitiger Lagerung um etwa 30% verdampft, so daß der erste Sprühstoß anstelle der klinisch erforderlichen Menge von etwa 50 mg nur 35 mg der Wirkstofflösung enthält. Dieser Verlust an Wirkstofflösung ist für ein Arzneimittel, das zur sofortigen Behandlung akuter Angina-pectoris-Anfälle eingesetzt wird, zu groß und dessen Einsatz damit therapeutisch nicht vertretbar. Durch die Verflüchtigung des Ethylalkohols kann ferner auch eine Verstopfung der Ventile hervorgerufen werden, die eine dosierte Anwendung völlig verhindert. Der hohe Gehalt an C₂- bis C₄-Alkohol und vorzugsweise an Ethylalkohol hat bei der lingualen Anwendung noch den Nachteil, daß die Schleimhaut im lingualen Bereich zu stark angeregt wird und der Plasmaspiegel gegenüber gleichwertigen in der Therapie seit Jahrzehnten bewährten Präparaten sehr schnell zu hoch ansteigt. Dieses kann zu vasomotorischen Kopfschmerzen, orthostatischen Dysregulationen, Schwächegefühl oder Benommenheit führen. Darüber hinaus sind Arzeimittel mit hochkonzentrierten Alkoholmengen für bestimmte Patientengruppen (z.B. Schwangere, Alkoholabhängige, Leber- oder Nierengeschädigte) ungeeignet und ergeben bei der Herstellung und Lagerung erhebliche Sicherheitsprobleme.

Abgesehen von dem zu hohen Ethylalkoholgehalt dieses treibgasfreien Aerosol-Präparates wird als Trägerstoff ein Polyalkylenglykol oder ein C₂- bis C₈-Polyhydroxyalkohol vorgeschlagen, die meist schleimhautreizend wirken oder toxikologisch bedenklich sind.

Aufgabe der Erfindung ist es, einen Nitroglycerin-Pumpspray vorzuschlagen, dessen flüssige Phase so beschaffen ist, daß bei der geforderten sofortigen wirksamen Behandlung akuter lebensbedrohlicher Zustände der erste Sprühstoß auch noch nach Lagerung die erforderliche Wirkstoffmenge sofort abgibt, und bei dem die therapeutisch ausreichenden Plasmaspiegel an Nitroglycerin nicht erheblich überschritten werden.

Zur Lösung dieser Aufgabe wird demzufolge ein Nitroglycerin-Pumpspray gemäß Hauptanspruch vorgeschlagen, wobei besondere Ausführungsformen in den Unteransprüchen erwähnt sind.

Überraschenderweise hat sich gezeigt, daß ein Pumpspray mit der flüssigen Phase aus 10 bis 40 Gew.% Ethylalkohol und 90 bis 60 Gew.% eines Neutralöles noch einen gleichmäßigen Sprühnebel ergibt und daß nach längerer Lagerzeit noch eine ausreichende Menge an dem Präparat bzw. an dem Wirkstoff zur Verfügung steht. Selbst wenn bei einer Lagerung nach 7 Tagen beim ersten Sprühstoß ein Mengenverlust von max. 10%, bezogen auf die in der Dosierkammer befindliche Wirkstofflösung, zu verzeichnen ist, so ist der Verlust an dem Wirkstoff geringer, weil dieser in dem schwerer verdampfbaren Neutralöl gelöst bleibt.

Bei dem erfindungsgemäßen Nitroglycerin-Pumpspray wird das Nitroglycerin in einer etwa 5%igen öligen Lösung vorgegeben und dann mit den weiteren erforderlichen Mengen Neutralöl und gegebenenfalls mit weiteren Zusatzstoffen vermischt und unter Zugabe von Ethylalkohol in die Pumpspray-Flaschen abgefüllt.

Als Neutralöle können alle fetten Öle verwendet werden. Bevorzugt werden jedoch synthetische Triglyceride, deren Fettsäureanteil aus gesättigten C₈- bis C₁₂-Fettsäuren besteht. Diese Triglyceride werden auch als Miglyol-Typen bezeichnet. Hierbei unterscheidet man nach dem Gehalt an Caprylsäure (C_{8:0}) und Caprinsäure (C_{10:0}) verschienede Miglyöl-Typen.

Zum Beispiel enthält das Miglyol 812
CS : 50 - 65 %
CN : 30 - 45 %,
während das Miglyol 810
CS : 65 - 75 %
CN : 25 - 35 %
enthält.

Es können aber auch natürliche Öle eingesetzt werden, wobei allerdings solche Öle bevorzugt sind, die möglichst wenig ungesättigte Fettsäuren enthalten. Dies kann bei Ölen, die einen hohen Gehalt an ungesättigten Fettsäuren besitzen, durch Hydrierung erreicht werden. Der Anteil des Neutralöls im erfindungsgemäßen Nitroglycerin-Pumpspray beträgt 90 bis 60 Gew.%, liegt aber gewöhnlich bei 85 bis 70 Gew.%; bevorzugt sind Neutralölmengen von etwa 80 Gew.%.

Der Nitroglycerinanteil im erfindungsgemäßen Spray kann variieren, liegt aber gewöhnlich zwischen 0,1 bis 2 Gew.%. Bevorzugt sind Nitroglyceringehalte von 0,6 bis 0,9 Gew.%, wie z.B. 0,8 Gew.%.

Außer den zuvor genannten Hauptbestandteilen kann der erfindungsgemäße Nitroglycerin-Pumpspray übliche Zusätze wie Geschmacksstoffe enthalten, beispielsweise Pfefferminzöl oder Menthol, die dem Spray einen erfrischenden Geschmack verleihen. Die Geschmacksstoffe sind meist in einer Menge von etwa 0,1 bis 1 Gew.% und vorzugsweise 0,4 bis 0,8 Gew.% vorhanden. Die Ausbringungsmengen betragen bei Anwendung des Sprays je Sprühstoß etwa 25 bis 100 mg und vorzugsweise 50 mg.

Im folgenden soll die Erfindung anhand von Beispielen näher erläutert werden.

### Beispiel 1

Es wurde ein Nitroglycerin-Pumpspray mit der folgenden Zusammensetzung hergestellt:

| | |
|---|---|
| Nitroglycerin 5%ig in Miglyol 812 (WZ) | 20,1 Gew.% |
| Ethanol | 20,0 Gew.% |
| Miglyol 812 (WZ) | 59,2 Gew.% |
| Krauseminzöl | 0,7 Gew.% |

Nach 7-tägiger Lagerung bei Raumtemperatur betrug die prozentuale Abweichung der Ausbringungsmenge des ersten Sprühstosses vom Sollwert 10%.

Bei einem Vergleichspräparat gemäß Beispiel 1 der DE-OS 39 22 650 betrug die Abweichung der Ausbringungsmenge beim ersten Sprühstoß 33% vom Sollwert. Dieses ist für ein Arzneimittel zur Anfallsbehandlung, bei dem der erste Sprühstoß schon eine genügende Menge an Wirkstofflösung zur Verfügung stellen muß, zu groß.

### Beispiel 2

Es wurde ein Nitroglycerin-Pumpspray analog Beispiel 1 hergestellt, wobei jedoch jetzt anstelle der 59,2 Gew.% Miglyol 812 die gleiche Menge Rüböl und in einem weiteren Ansatz die gleiche Menge Miglyol 810 eingesetzt wurden. In beiden Fällen verhielt sich das Präparat wie das des Beispiels 1; es bildete sich ein gleichmäßiger Sprühnebel. Die Ausbringungsmenge nach 7-tägiger Lagerung betrug beim ersten Sprühstoß etwas mehr als 90%, bezogen auf den Sollwert.

## Patentansprüche

1. Nitroglycerin-Pumpspray mit einem Gehalt an 0,2 bis 3,5 Gew.% Nitroglycerin und bis zu 3 Gew.% üblicher Zusatzstoffe wie Geschmacksstoff oder Antioxydantien sowie einer flüssigen Phase aus aliphatischen C₂- bis C₄-Alkoholen und einem Trägerstoff, **dadurch gekennzeichnet,** daß die flüssige Phase aus 10 bis 40 Gew.% Ethylalkohol und 90 bis 60 Gew.% eines Neutralöles besteht.

2. Nitroglycerin-Pumpspray nach Anspruch 1, **dadurch gekennzeichnet,** daß die flüssige Phase aus 15 bis 30 Gew.% Ethylalkohol und 85 bis 70 Gew.% eines Neutralöles besteht.

3. Nitroglycerin-Pumpspray nach Anspruch 1, **dadurch gekennzeichnet,** daß die flüssige Phase etwa 20 Gew.% Ethylalkohol und etwa 80% eines Neutralöles besteht.

4. Nitroglycerin-Pumpspray nach Anspruch 1 bis 3, dadurch gekennzeichnet, daß das Neutralöl ein gesättigtes natürliches Öl wie Rüböl oder ein C₈- bis C₁₂-Fettsäuretriglycerid ist.

## Claims

1. Nitroglycerine pump spray containing 0.2 to 3.5 % by wt. nitroglycerine and up to 3 % by wt. usual additives such as flavourings or antioxidants and a liquid phase comprising aliphatic C₂ to C₄ alcohols and a carrier, characterized in that the liquid phase consists of 10 to 40 % by wt. ethyl alcohol and 90 to 60 % by wt. of a neutral oil.

2. Nitroglycerine pump spray according to claim 1, characterized in that the liquid phase consists of 15 to 30 % by wt. ethyl alcohol and 85 to 70 % by wt. of a neutral oil.

3. Nitroglycerine pump spray according to claim 1, characterized in that the liquid phase consists of approximately 20 % by wt. ethyl alcohol and approximately 80 % of a neutral oil.

4. Nitroglycerine pump spray according to claims 1 to 3, characterized in that the neutral oil is a saturated natural oil such as rapeseed oil or a C₈ to C₁₂ fatty acid triglyceride.

## Revendications

1. Composition de nitroglycérol à pulvériser en atomiseur à pompe, ayant une teneur en nitroglycérol de 0,2 à 3,5 % en poids et une teneur en additifs usuels, tels qu'aromatisant ou antioxydants, allant jusqu'à 3 % en poids, et comportant une phase liquide composée d'alcools aliphatiques en C₂-C₄ et un véhicule, caractérisée en ce que la phase liquide consiste en 10 à 40 % en poids d'alcool éthylique et 90 à 60 % en poids d'une huile neutre.

2. Composition de nitroglycérol à pulvériser en atomiseur à pompe selon la revendication 1, caractérisée en ce que la phase liquide consiste en 15 à 30 % en poids d'alcool éthylique et 85 à 70 % en poids d'une huile neutre.

3. Composition de nitroglycérol à pulvériser en atomiseur à pompe selon la revendication 1, caractérisée en ce que la phase liquide consiste en environ 20 % en poids d'alcool éthylique et environ 80 % en poids d'une huile neutre.

4. Composition de nitroglycérol à pulvériser en atomiseur à pompe selon les revendications 1 à 3, caractérisée en ce que l'huile neutre est une huile naturelle saturée telle que l'huile de colza ou un triglycéride d'acides gras en C₈-C₁₂.
